# EUROPÄISCHE PATENTANMELDUNG

(11) **EP 2 025 747 A1**
(43) Veröffentlichungstag der Anmeldung: **18.02.2009**
(21) Anmeldenummer: 07114342.4
(22) Anmeldetag: 14.08.2007
(51) Int. Cl.: C12N 5/08

(54) **Verfahren zur in-vitro-Amplifikation von regulatorischen T-Zellen**

(71) Anmelder: TumorTec GmbH, 50933 Köln (DE)
(72) Erfinder: Hombach, Andreas, 50825 Köln (DE); Abken, Hinrich, 56414 Meudt (DE)
(74) Vertreter: von Kreisler Selting Werner

(57) **Zusammenfassung**

Die Erfindung betrifft ein Verfahren zur *in vitro* Vermehrung (Amplifikation) von regulatorischen T-Zellen (Treg-Zellen), bevorzugt humanen Treg-Zellen, für die Anwendung in der Biotechnologie und Therapie von Erkrankungen. Weiterhin werden geeignete Agentienzusammensetzungen für solch ein Verfahren zur Verfügung gestellt.

## Beschreibung

Die Erfindung betrifft ein Verfahren zur *in vitro* Vermehrung (Amplifikation) von regulatorischen T-Zellen (Treg-Zellen), bevorzugt humanen Treg-Zellen, für die Anwendung in der Biotechnologie und Therapie von Erkrankungen. Weiterhin werden geeignete Agentienzusammensetzungen für solch ein Verfahren zur Verfügung gestellt.

### Hintergrund der Erfindung

Das zelluläre Immunsystem besteht aus einer aktivierenden und einer supprimierenden Komponente. Während die aktivierenden Zellen Effektoren wie zytotoxische CD8⁺ T-Zellen, Helfer CD4⁺ T-Zellen, NK-Zellen u.a. umfassen, stellen regulatorische Suppressor T-Zellen (Treg-Zellen) einen Teil des supprimierenden Immunsystems dar. Regulatorische T-Zellen gehören der CD4⁺ T-Zell Population an und bestehen wiederum aus mehreren Subpopulationen. Die bisher am besten untersuchten "naturally occuring" Treg-Zellen sind an der Kontrolle der Immunantwort gegen Selbst-Antigene beteiligt und durch die besonders hohe Expression von CD25 sowie die Expression des nukleären Proteins FoxP3 gekennzeichnet (Sakaguchi, S. et al, J. Immunol. 155, 1151-1164 (1995); Suri-Payer, E. et al., J. Immunol. 160, 1212-1218 (1998); Dieckmann, D. et al., J. Exp. Med. 196, 247-253 (2002); Jonuleit, J. et al., J. Exp. Med. 193, 1285-1294 (2001); Sakaguchi, S., Cell 101, 455-458 (2000)). Jedoch sind diese Markerproteine nicht ausschließlich auf regulatorischen Zellen exprimiert, was eine funktionelle Definition regulatorischer Zellen notwendig macht. Ein typischer funktioneller Nachweis besteht darin, dass regulatorische T-Zellen unter definierten Bedingungen *in vitro* die Proliferation von autologen CD4⁺CD25⁻ T-Zellen supprimieren.

Regulatorische T-Zellen sind an der Limitierung einer ablaufenden Immunreaktion sowie an der Unterdrückung einer Autoimmunreaktion beteiligt. Funktionsstörungen sowie eine verminderte Anzahl der regulatorischen T-Zellen führen zu einer Vielzahl von ernsthaften Autoimmun-Erkrankungen, wie z.B. Typ-1 Diabetes (Kukreja, A. et al., J. Clin. Invest. 109, 131-140 (2002)), multipler Sklerose (Viglietta, V. et al., J. Exp. Med. 199, 971-979 (2004)), rheumatoider Arthritis (Ehrenstein, M. R. et al., J. Exp. Med. 200, 277-285 (2004)), autoimmun-polyglandulares Syndrom Typ II (Kriegel, M. A. et al., J. Exp. Med. 199, 1285-1291 (2004)). Bei diesen und zahlreichen weiteren Erkrankungen ist eine veränderte numerische wie funktionelle Balance zwischen regulatorischer T-Zellen und Effektor T-Zellen ursächlich an der Pathogenese beteiligt. Weiterhin sind Treg-Zellen an der Transplantat Toleranz (Wood, K. J., Sakaguchi, S., Nat. Rev. Immunol. 3, 199-210 (2003)), Tumor Immunität (Gallimore, A., Sakaguchi, S., Immunol. 107, 5-9 (2002)) und an der Immunabwehr und Persistenz von Infektionen (Belkaid, Y. et al., Nat. 420, 502-507 (2002); Suvas, S. et al., J. Exp. Med. 198, 889-901 (2003)) beteiligt.

In diesen Situationen wäre möglicherweise eine Applikation (Transfusion) von amplifizierten, aktivierten und autologen regulatorischen T-Zellen zur Unterdrückung der deregulierten, auto-aggressiven Immunreaktion von therapeutischem Nutzen. Die therapeutische Wirksamkeit zeigte sich in einem murinen Modell, in dem ex vivo amplifizierte Treg-Zellen die graft-versus-host disease (GvHD) nach Knochenmarktransplantation verhindern können (Taylor, P. A. et al., Blood 99, 3493-3499 (2002)). Jedoch sind bisher eine breitere Untersuchung derartiger Ansätze und eine klinische Anwendung an dem Fehlen eines effizienten Verfahrens zur *in vitro* Vermehrung funktioneller Treg-Zellen gescheitert.

Mehrere Untersuchungen legen nahe, dass *in vivo* regulatorische T-Zellen im Thymus generiert und sich unter physiologischen Bedingungen vermehren können. Die notwendigen Signale und exakten Voraussetzungen, unter denen Treg-Zellen proliferieren, sind jedoch nicht bekannt. Für eine erfolgreiche *in vitro* Amplifikation der Treg-Zellen kommt erschwerend hinzu, dass frisch isolierte Treg-Zellen *in vitro* anerg und hyporesponsiv sind, auch wenn die Zellen mit hohen Konzentrationen stimulierender Antikörper inkubiert werden (Shevach, E. H., et al., Immunol. Rev. 182, 58-67 (2001); Thornton, A. M., Shevach, E. M., J. Exp. Med. 188, 287-296 (1998)). Es sind mehrere Verfahren beschrieben, Treg-Zellen *in vitro* zu amplifizieren, jedoch ist diesen Verfahren gemeinsam, dass sie zu einer unzureichenden Anzahl von amplifizierten Zellen und/oder zu funktionell eingeschränkt aktiven Treg-Zellen führen.

Durch Zugabe von IL-2 gelingt es, Treg-Zellen nach Stimulation mit Antigenbeladenen Antigen-präsentierenden Zellen (APC) oder nach CD3 Stimulation zur Proliferation zu induzieren (Shevach, E. H., et al., Immunol. Rev. 182, 58-67 (2001); Thornton, A. M., Shevach, E. M., J. Exp. Med. 188, 287-296 (1998)). Dabei vermag die CD3 Stimulation in Gegenwart von IL-2 eine begrenzte Expansion muriner Treg-Zellen *in vitro* innerhalb von 7 Tagen zu induzieren (Takahashi, T. et al., Int. Immunol. 10, 1969-1980 (1998); Thornton A.M. et al., J. Immunol. 164, 183-190 (2000). Während diese amplifizierten Zellen anfänglich 4- bis 6-fach stärkere Suppression aufweisen als die frisch isolierten murinen Treg-Zellen (Takahashi, T. et al., Int. Immunol. 10, 1969-1980 (1998)), so verlieren sie doch ihre Suppressoraktivität nach mehrfachen Restimulationen (Chai J.G. et al., Eur. J. Immunol 32, 2365-2375 (2002 ).

Murine Treg-Zellen wurden in Gegenwart eines stimulierenden anti-CD3 Antikörpers und IL-2 *in vitro* für 10 Tage amplifiziert (Taylor, P. A. et al., Blood 99, 3493-3499 (2002)).

Tang, Q. et al., J. Exp. Med. 199, 1455-1465 (2004)) beschrieben ein Verfahren zur Amplifikation muriner T-Zellen mit Hilfe von Beads, an die anti-CD3 und anti-CD28 Antikörper gekoppelt sind, sowie hohe Dosen von IL-2. Dieses Verfahren erlaubt eine 200fache Amplifikation der Treg-Zellen innerhalb von 2 Wochen.

Ein super-agonistischer anti-CD28 Antikörper wurde zur Amplifikation von T-Zellen, u.a. auch Treg-Zellen, in der Rag2 -/-, gammac -/- Maus in vivo genutzt (Legrand, J. et al., Blood 108, 238-245 (2006)). Der verwendete superagonistische anti-CD28 Antikörper führt zu einer T-Zell Aktivierung ohne Beteiligung des T-Zell Rezeptors (TCR) und vermochte im Tierversuch murine Treg-Zellen in vivo unter Erhalt der funktionellen Aktivität zu vermehren, so dass eine Autoimmunerkrankung nach adoptivem Transfer der amplifizierten Treg-Zellen unterdrückt werden konnte (Beyersdorf, N. et al., J. Exp. Med. 202, 445-455 (2005)).

Auch wenn diese Verfahren die Amplifikation muriner Treg-Zellen hinlänglich erlauben, so stellt die Amplifikation humaner CD4⁺CD25⁺ Treg-Zellen ein größeres Problem dar. Humane Treg-Zellen sind im peripheren Blut in noch geringerer Zahl vorhanden als murine Treg-Zellen und stellen nur 1-2% der CD25^{high} Zellen dar (Baecher-Allan, C. et al., J. Immunol. 167, 1245-1253 (2001)). Weiterhin ist die Suppressor-Funktion der humanen Treg-Zellen weniger stark ausgeprägt als die der murinen Treg-Zellen. Dieses zusammen macht eine noch leistungsfähigere Treg Zell Amplifikation notwendig. Bisherige Versuche, humane Treg-Zellen *in vitro* zu amplifizieren oder zu aktivieren, erwiesen sich als völlig unzureichend und führten kaum zu einer nennenswerten Ausbeute an funktionell aktivierten Zellen. Erschwert wird die Entwicklung eines effizienten Verfahrens dadurch, dass die Voraussetzungen für eine effiziente Stimulation humaner Treg-Zellen nicht hinlänglich bekannt sind. Die derzeitigen Vorstellungen von der Voraussetzung zur Amplifikation von funktionellen humanen Treg-Zellen gehen von einer Subpopulation dendritischer Zellen (DC) aus, die die Proliferation von Treg-Zellen in einem TGF-β abhängigen Prozess unterstützen (Ghiringhelli, F. et al., J. Exp. Med. 202, 9919-929 (2005)). Bisherige Verfahren nutzen deswegen DC Zellen in Gegenwart einer hohen Konzentrationen von IL-2, um die Treg-Zellen zu amplifizieren (Yamazakti S. et al., Proc. Natl. Acad. Sci USA 103, 2758-2763 (2006); Setoguchi R. et al., J. Exp. Med. 201, 723-735 (2005); Hoffmann, P. et al., Blood 104, 895-903 (2004)).

Kurzzeitige Antigen-spezifische Ansätze mit DC und IL-2 oder IL-7 Gabe führten jedoch nur zu einer sehr begrenzten Proliferation der Treg-Zellen (3fache Amplifikation in 7 Tagen), mehrfache Restimulierungen verbesserte nicht das Ergebnis (Jiang, S. et al., Blood 102, 2180-2186 (2003)). Weiterhin sind Verfahren beschrieben, die allogene mononukleäre Zellen und lymphoblastoide Feeder Zellen sowie agonistische anti-CD3 Antikörper und IL-2 verwenden, was eine 20fache Amplifikation in 14 Tagen ermöglicht (Levings, M. K. et al., J. Exp. Med. 193, 1295-1302 (2001)). Nachteil dieser Verfahren ist jedoch die Verwendung von allogenen oder autologen Stimulatorzellen, was einerseits eine Rückisolierung amplifizierter Treg-Zellen notwendig macht, andererseits einen erheblichen Aufwand in der Bereitstellung der jeweiligen Stimulatorzellen darstellt.

Hoffmann, P. et al., Blood 104, 895-903 (2004)) beschrieben ein Verfahren zur Amplifikation von humanen Treg-Zellen, das auf der Verwendung "künstlicher Antigen präsentierender Zellen" (artificial APCs) Zellen in der Gegenwart hoher Dosierung von IL-2 beruht und eine bis zu 40.000fache Amplifikation der Treg-Zellen in 3 bis 4 Wochen ermöglicht. Als "künstliche Antigen präsentierende Zellen" dienen Beads, die mit anti-CD3 und anti-CD28 Antikörper beschichtet sind. Alternativ können auch L929 Fibroblasten als Feeder verwendet werden, die den Fc Rezeptor FcγRII (CD32) exprimieren und nach Bindung die anti-CD3 und anti-CD28 Antikörper den Treg-Zellen präsentieren (Hoffmann, P. et al., Blood 104, 895-903 (2004)). Dabei scheint die selektive Expansion von CD45RA⁺ naiven Treg-Zellen die beste Strategie darzustellen (Hoffmann, P. et al., Blood 108, 4260-4267 (2006)). Gemeinsam ist diesen Verfahren jedoch eine hohe Dosis IL-2.

Ein weiteres derzeitig meist angewendetes Verfahren beruht auf der Verwendung von Beads, die mit anti-CD3 und anti-CD28 Antikörpern beschichtet sind, Zusatz von IL-2 sowie bestrahlten CD4⁺CD25⁻ Feeder Zellen, die im Verhältnis 1:1 den regulatorischen Zellen zugesetzt werden (Godfrey, W. R. et al., Blood, 104, 453-461 (2004)). Dadurch konnte eine bis zu 100fache Amplifikation der Treg-Zellen in 21 Tagen erreicht werden. Erhebliche Probleme bei diesem Verfahren ergeben sich jedoch dadurch, dass sowohl die Beads als auch die Feeder Zellen die Kultur amplifizierter Treg-Zellen in erheblicher Anzahl verunreinigen, was aufwendige Reinigungsschritte der Treg-Zellen mit möglichem Zell- und Funktionsverlust notwendig macht. Insbesondere bei der Verwendung der Antikörper-beschichteten Beads erwies sich deren vollständige Abreicherung als kaum möglich, was eine therapeutische oder biotechnologische Verwendung der amplifizierten Treg-Zellen erheblich einschränkt oder unmöglich macht.

Gesucht wird ein Verfahren zur Amplifikation von regulatorischen T-Zellen, insbesondere von humanen Treg-Zellen, das eine annähernd unbegrenzte Amplifikation ermöglicht und funktionell aktive Treg-Zellen bereitstellt, wobei die Nachteile der Kontamination durch Feeder Zellen und Beads in dem Amplifikationsprozess vermieden werden.

### Kurzbeschreibung der Erfindung

Es wurde nun ein sehr effizientes Amplifikationsverfahren funktionell aktiver regulatorischer T-Zellen, bevorzugt humaner Treg-Zellen, gefunden, bei dem Treg-Zellen auf einer Oberfläche ohne IL-2-Zugabe inkubiert werden, die mit einem agonistischen Agens mit Spezifität für CD3 oder TCR und mit einem agonistischem Agens für CD28, bevorzugt agonistischen Antikörpern, beschichtet sind. Dieses Verfahren erlaubt die annähernd unbegrenzte Amplifikation funktioneller Treg-Zellen in Reinform. Die Erfindung betrifft somit
(1) ein Verfahren zur Amplifikation von regulatorischen T-Zellen (nachfolgend "Treg-Zellen"), umfassend das Inkubieren von Ausgangs-Treg-Zellen in Gegenwart wenigstens eines ersten agonistischen (d.h. stimulierenden) Agens mit Spezifität für CD3 oder TCR und und wenigstens eines zweiten agonistischen (stimulierenden) Agens mit Spezifität für CD28, wobei das erste und zweite agonistische Agens auf einer Oberfläche gebunden ist und das Inkubieren ohne IL-2 erfolgt;
(2) eine im Wesentlichen planare Oberfläche, die mit wenigstens einem agonistischen, für CD3 oder TCR spezifischen Agens und wenigstens einem agonistischen, für CD28 spezifischen Agens beschichtet ist; und
(3) die Verwendung einer wie vorstehend unter (2) definierten im Wesentlichen planaren Oberfläche zur Amplifikation von regulatorischen T-Zellen.

### Figuren

Fig. 1: Isolierung und Charakterisierung humaner CD4⁺CD25⁺ regulatorischer T Zellen aus dem peripheren Blut.
Fig. 2: Suppressor Aktivität von isolierten humane CD4⁺CD25⁺ Treg-Zellen.
Fig. 3: Amplifikation von humanen Treg-Zellen auf Oberflächen, die mit agonistischen, CD3 und CD28 spezifischen Antikörpern beschichtet sind.
Fig. 4: Nachweis der Proliferation der Treg-Zellen auf beschichteten Oberflächen.
Fig. 5: Zytokin Sekretion der CD4⁺CD25⁺ und CD4⁺CD25⁻ T-Zell Populationen nach Inkubation auf beschichteten Oberflächen.
Fig. 6: Amplifikation der CD4⁺CD25⁺ T-Zellen auf beschichteten Oberflächen.
Fig. 7: Amplifizierte Treg-Zellen behalten ihre Suppressoraktivität.

### Detailierte Beschreibung der Erfindung

Bei dem erfindungsgemäßen Verfahren (1) der Erfindung werden Treg-Zellen auf einer Oberfläche inkubiert, die wenigstens mit einem agonistischen Agens mit Spezifität für CD3 oder TCR und mit einem agonistischen Agens für CD28, bevorzugt agonistische Antikörper, beschichtet sind. Die agonistischen Agentien sind vorzugsweise agonistische Antikörper. Im Sinne des erfindungsgemäßen Verfahrens können dabei Oberflächen eingesetzt werden, die mit folgenden Kombinationen an agonistischen Agens beschichtet sind: CD3/CD28, TCR/CD28 und CD3/TCR/CD28, wobei Kombinationen mit nur einem ersten agonisten Agens bevorzugt sind
"Treg-Zellen" im Sinne der vorliegenden Erfindung umfassen dabei CD4⁺CD25⁺,CD4⁺CD25^{high} und CD4⁺CD25^{high} Fox P3+ Zellen.

Bei dem erfindungsgemäßen Verfahren kann die Oberfläche jegliche dreidimensionale Struktur aufweisen. Es hat sich jedoch als vorteilhaft erwiesen, wenn im Wesentlichen planare Oberflächen, d. h. Mikrotiterplatten, Kulturwannen, - flaschen und dergleichen als Träger für die Agentien eingesetzt werden, so dass die beschichtete Oberfläche gleichzeitig den Kulturrahmen definiert. Besonders bevorzugt kommen Polymeroberflächen wie Polystyrol-Oberflächen, die mit agonistischen anti-CD3 und anti-CD28 Antiköpern beschichtet sind, zurAnwendung. Die Oberfläche kann vor der Beschichtung mit agonistischen Agens aktiviert oder mit einer aktiven Schicht überzogen werden. Besonders bevorzugte Oberflächen im Sinne der vorliegenden Erfindung sind PolySorb^{™} und MaxiSorb^{™} Mikrotiterplattern (Nunc, Wiesbaden, Deutschland), TC Mikrotiterplatten (Nunc, Wiesbaden, Deutschland, geeignet für die Zellkultur) und Polystyrol-Bakterienplatten (Sarstedt, Nümbrecht, Deutschland, geeignet für die Mikrobiologie). Auch jegliche andere Oberflächen, die mit derartigen Agentien beschichtet sind, können für dieses Verfahren genutzt werden. Die Beschichtung führt zur stabilen Verknüpfung der Antikörper mit der Oberfläche.

Bevorzugter anti-CD3 Antikörper für das erfindungsgemäße Verfahren ist der monoklonale Antikörper OKT3 (ATCC CRL 8001), bevorzugter anti-CD28 Antikörper der monoklonale Antikörper 15E8 (Van Lier, R. A. W. et al., Leukocyte Typing, Vol. IV, Ed. W. Knapp, Oxford University Press, Oxford, pp. 353-355 (1989)). Jeglicher andere agonistische anti-CD3, anti-TCR oder anti-CD28 Antikörper ist ebenfalls in dem erfindungsgemäßen Verfahren einsetzbar. Auch ist ein superagonistischer Antikörper allein in dem Verfahren verwendbar. An der Stelle von Antikörpern können auch andere agonistische, stimulierende Moleküle mit Spezifität für CD3 oder TCR sowie CD28 verwendet werden, z.B. Peptide, Aptamere, MHC-Peptid Komplexe oder Naturstoffe.

Als Inkubationsmedium im Sinne der vorliegenden Erfindung kann RPMI Medium mit fötalem Kälberserum oder mit Kälberserum verwendet werden. Auch können DMEM (Dulbecco's MEM). Iscove's modifiziertes MEM und serumfreie Kulturmedien wie Nutridoma (Roche) eingesetzt werden. RPMI-1640-Medium mit 10% (v/v) fötalem Kälberserum ist jedoch besonders bevorzugt. Die Kulturbedingungen in dem erfindungsgemäßen Verfahren sind vorzugsweise 34-38 °C Inkubationstemperatur und eine Luftfeuchte von 10-100%. Die Dauer der Inkubation richtet sich danach, um welchen Faktor die Zellen amplifiziert werden sollen. Sie beträgt vorzugsweise 1 Tag bis 5 Tage, bei einer längeren Inkubationszeit ist ein Wechsel auf eine neue, frisch beschichtete Oberfläche zu empfehlen.

Das erfindungsgemäße Verfahren (1) der Erfindung bedarf keiner weiteren stimulatorischen Additive wie Zytokine, insbesondere IL-2, Beads oder Feeder Zellen. Bei dem erfindungsgemäßen Verfahren erübrigt sich die Rück-Isolierung der amplifizierten Treg-Zellen von störenden Stimulatorzellen oder Beads, wie es bei bisher bekannten Verfahren notwendig war.

Die erfindungsgemäß amplifizierten Treg-Zellen können wiederum auf beschichtete Oberflächen ausgesät und erneut amplifiziert werden, ohne dass die Zellen ihre Teilungsfähigkeit einbüßen. Es konnte gezeigt werden, dass dieses Verfahren ohne Einbuße der Funktion der erhaltenen Treg-Zellen mindestens 5fach wiederholt werden kann, jedoch sind weitere Amplifikationsansätze anschließend möglich, so dass eine annähernd unbegrenzte serielle Amplifikation erzielt werden kann.

Die Treg-Zellen behalten bei der erfindungsgemäßen Amplifikation ihren funktionell aktiven Zustand bei. Insbesondere vermögen derartig amplifizierte Treg-Zellen autologe CD4⁺CD25⁻ T-Zellen in ihrer Proliferation zu supprimieren. Sie sind darüber hinaus für eine Vielzahl von pharmakologischen Zwecken wie z. B. die adaptive Immuntherapie einsetzbar.

Die vorliegende Erfindung wird anhand der nachfolgenden Beisbiele näher erläutert. Diese schränken jedoch das Patentbegehren in keiner Weise ein.

### Beispiel und detaillierte Beschreibung der Figuren

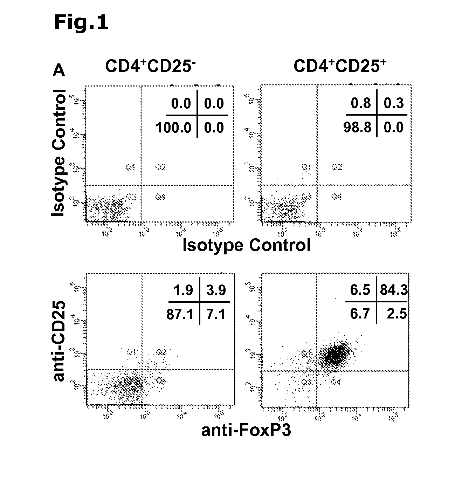

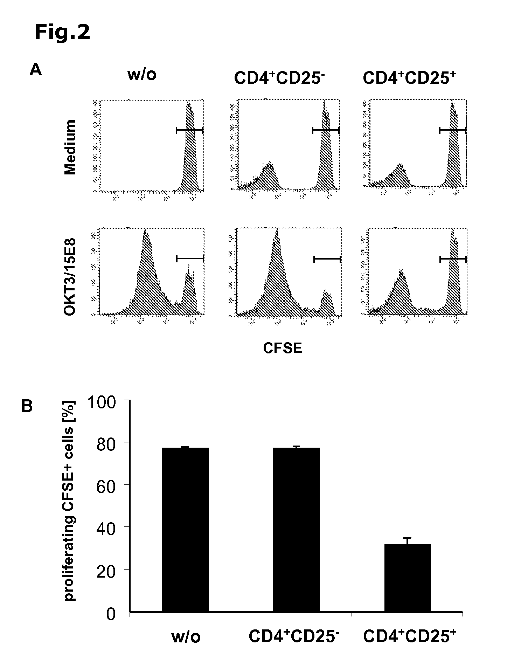

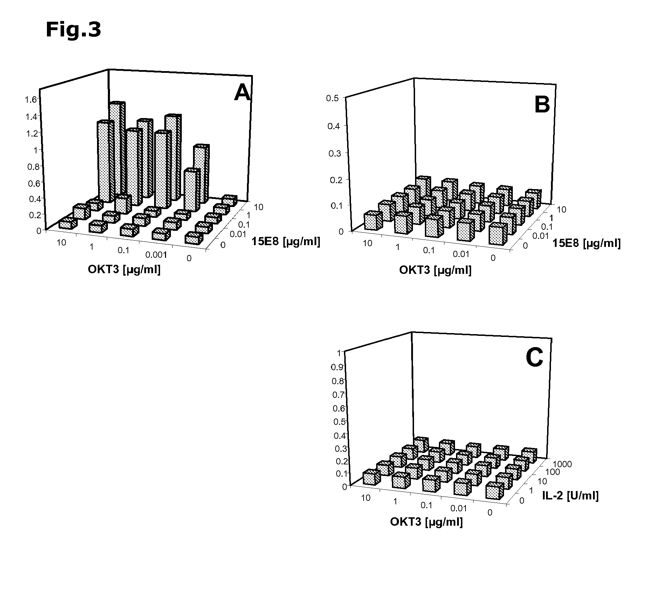

Beispiel: Amplifikation funktionell aktiver, humaner CD4⁺CD25^{high} Treg-Zellen. Mikrotiter 96-Loch Platten (Polysorb™, Nunc, Wiesbaden,Deutschland) wurden über Nacht mit dem anti-CD3 Antikörper OKT3 und dem anti-CD28 Antikörper 15E8 in Beschichtungspuffer (1,7 ml 0,2 M Na₂CO₃, 0,8 ml NaHCO₃ ad 10 ml aq. bidest) beschichtet. Die Platten wurden zweimal in PBS (8,0 g/l NaCl; 0,2 g/l KCl; 1,15 g/l Na₂HPO₄; 0,2 g/l KH₂PO₄; pH 7,4) gewaschen. Humane CD4⁺CD25^{high} Treg-Zellen wurden aus dem peripheren Blut isoliert (Fig. 1) und deren funktionelle Aktivität mit Hilfe eines Standard Suppressor Tests bestätigt (Fig. 2). Isolierte humane CD4⁺CD25^{high} Treg-Zellen (2.5 x 10⁴ Zellen/Loch) wurden in RPMI-1640-Medium, 10 % (v/v) fötales Kälberserum auf dieser beschichteten Oberfläche inkubiert. Die Proliferation der Treg-Zellen wurde mit Hilfe des Einbaus von BrdU unter Verwendung des "Cell Proliferation ELISA, BrdU colorimetric" (Roche, Mannheim,Deutschland) bestimmt. Fig. 3A zeigt eine typische Amplifikation der Treg-Zellen in Abhängigkeit von der Konzentration der beschichtenden Antikörper. Wenn die Zellen dagegen auf unbeschichteten Oberflächen, aber in Gegenwart der agonistischen Antikörper gelöst im Medium inkubiert werden, wird keine Treg Zell Proliferation induziert (Fig. 3B). Auch erfolgt keine Proliferation, wenn die Treg-Zellen auf anti-CD3 Antikörper OKT3 beschichteten Oberflächen in Gegenwart von IL-2 inkubiert werden (Fig. 3C). Nur unter den erfindungsgemäßen Bedingungen Fig. 3A werden die Treg-Zellen amplifiziert.

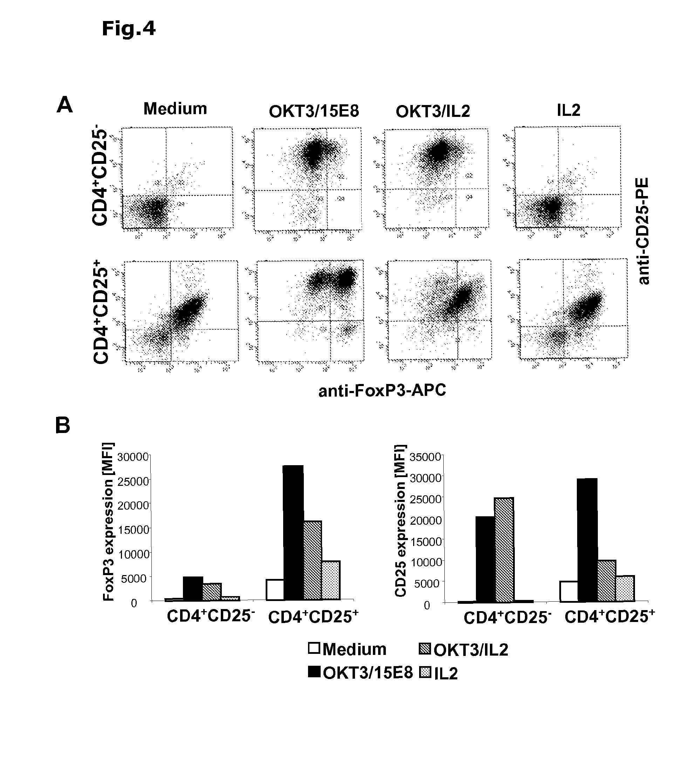

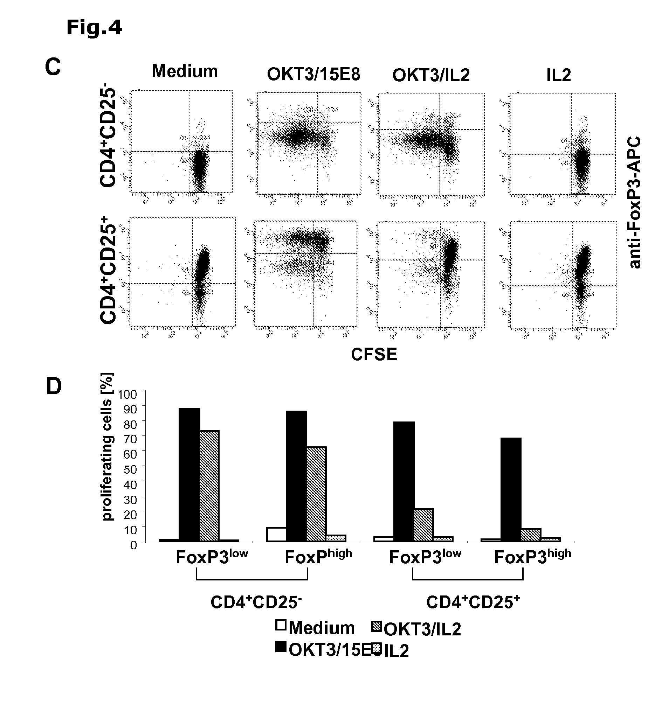

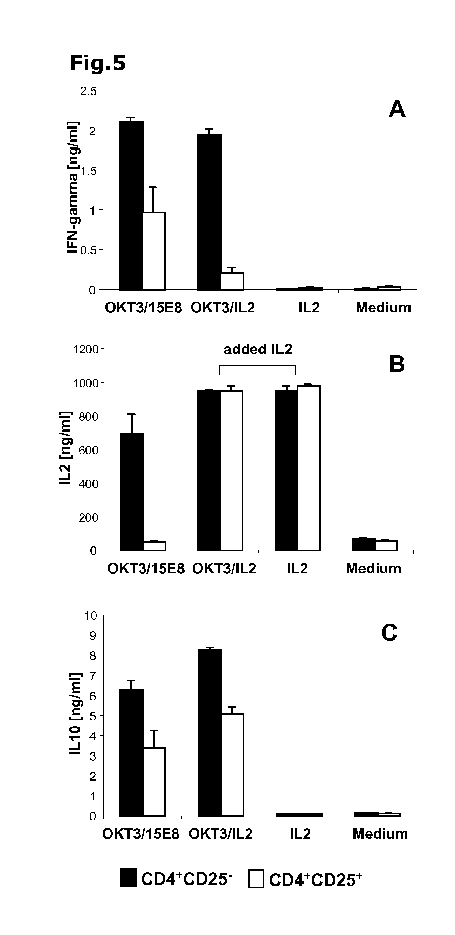

Zum Nachweis, dass die CD4⁺CD25⁺ regulatorischen T-Zellen auf dieser Oberfläche proliferieren und nicht möglicherweise kontaminierende CD4⁺CD25⁻ T-Zellen, wurden Untersuchungen mit Hilfe von CSFE-markierten Zellen durchgeführt (Fig. 4). Hierbei zeigte sich, dass die CD4⁺CD25⁺FoxP3⁺ Treg-Zellen und nicht die CD4⁺CD25⁻ T-Zellen unter diesen Bedingungen proliferieren. Weiterhin ist das Muster der Zytokine im Kulturüberstand der amplifizierten Zellen charakteristisch für Treg-Zellen, das sich deutlich von den sezernierten Zytokinen der CD4⁺CD25⁻ T-Zellen unterscheidet (Fig. 5).

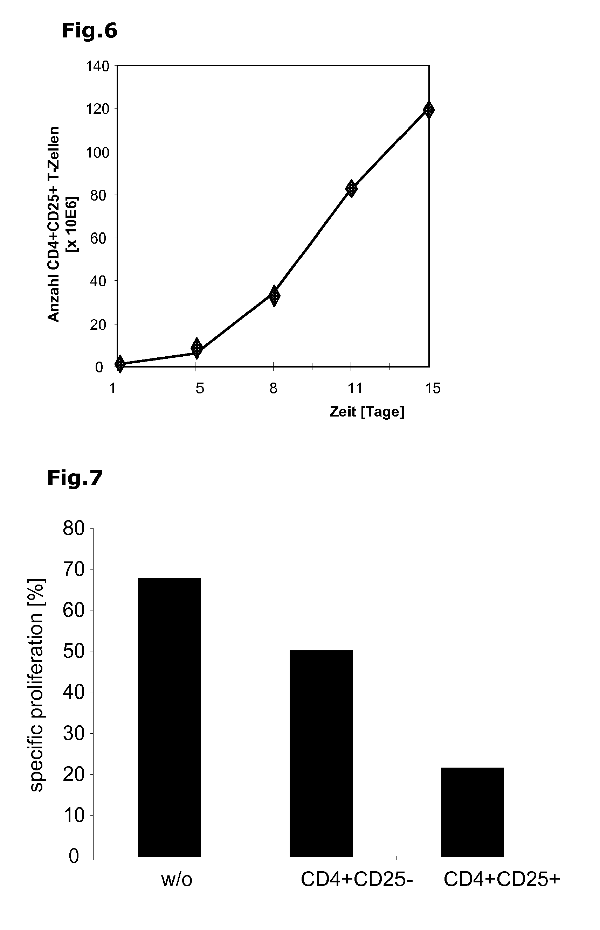

Fig. 6 zeigt beispielhaft die numerische Amplifikation der Treg-Zellen nach Inkubation auf den erfindungsgemäßen Oberflächen. Ausgehend von 1x10⁶ CD4⁺CD25⁺ T-Zellen wurden innerhalb von 15 Tagen ca. 1,2 x 10⁸ CD4⁺CD25⁺ T-Zellen erhalten, was eine 120fache Vermehrung der Treg-Zellen entspricht. Fig. 7 zeigt, dass die amplifizierten Treg-Zellen ihre Suppresor-Aktivität beibehalten haben.

### Detaillierte Beschreibung der Figuren

Fig. 1: Isolierung und Charakterisierung humaner CD4⁺CD25⁺ regulatorischer T Zellen aus dem peripheren Blut.

Periphere Blut-Lymphozyten gesunder Spender wurden mit Hilfe der Dichtezentrifugation gewonnen und Monozyten durch Pastik-Adhärenz depletiert. Nicht-adhärente Lymphozyten wurden in kaltem PBS, 0,5 % (w/v) BSA, 1 % (v/v) FCS und 2 mM EDTA gewaschen. CD4⁺CD25⁺ Treg-Zellen und CD4⁺CD25⁻ T Zellen wurden durch magnetisch aktivierte Zellsortierung (MACS) unter Verwendung des "CD25 T cell isolation kit" (Miltenyi Biotech, Bergisch Gladbach) isoliert, wobei 5 µL anti-CD25 Beads pro 10⁷ Lymphozyten verwendet wurden. Wir erhielten in einem typischen Ansatz 1 - 3 x 10⁶ CD4⁺CD25⁺ T Zellen aus 2 x 10⁸ CD4⁺ T-Zellen. Die Reinheit der isolierten CD4⁺CD25⁺ Treg-Zellen und CD4⁺CD25⁻ T Zellen wurde mit Hilfe der Durchflusszytometrie hinsichtlich der Expression von CD25 (PE-conjugated anti-CD25 mAb, Miltenyi Biotech, Bergisch Gladbach, Deutschland) und FoxP3 ("FoxP3 labeling kit", APC-conjugated anti-human FoxP3 mAb PCH101 Natutec, Frankfurt, Deutschland) analysiert. Färbungen mit Isotyp Antikörpern dienten als Kontrolle für die Spezifität der verwendeten Antikörper. Die Reinheit der isolierten CD25⁺FoxP3⁺ T-Zellen war typischerweise > 80 %. Die Zahlen im Quadranten geben die Anzahl Zellen von der Gesamtzellzahl (%) an. Isolierte T Zellen wurde gewaschen, gezählt und für die in vitro Amplifikation eingesetzt.

Fig. 2: Isolierte humane CD4⁺CD25⁺ Treg-Zellen zeigen Suppressor Aktivität.

Frisch isolierte CD4⁺CD25⁻ T Zellen (1 x 10⁷ Zellen/ml) wurden mit 5-Carboxylfluorescein diacetate succinimidyl ester (CFSE) (1 µM) (Molecular Probes, Göttingen, Deutschland) markiert und 5 x1 0⁴ Zellen/Loch entweder mit nichtmarkierten CD4⁺CD25⁻ und CD4⁺CD25⁺ T Zellen (5 x 10⁴ Zellen/Loch) oder allein (1 x 10⁵ Zellen/Loch) in Gegenwart der gelösten anti-CD3 Antikörper OKT3 (10 µg/ml) und anti-CD28 mAb 15E8 (1 µg/ml) in RPMI-1640-Medium, 10 % (v/v) FCS inkubiert. Nach 6 Tagen wurden die proliferierenden und nicht-proliferierenden CFSE-markierten Zellen mit Hilfe der Druchflusszytometrie durch Messung der Abnahme der CSFE Markierung bestimmt. Die Anzahl proliferierender Zellen [%] wurde wie folgt bestimmt: (1 - nicht-proliferierende, CFSE-markierte Zellen [%] unter Stimulationsbedingungen / nicht-proliferierende CFSE-markierte Zellen [%] unter nicht-stimulierenden Bedingungen) x 100. Der Test wurde als Dreifach Ansatz durchgeführt und der Mittelwert bestimmt. (A) zeigt typische Histogramme, (B) die numerische Auswertung eines typischen Ansatzes.

Fig. 3: Amplifikation von humanen Treg-Zellen auf Oberflächen, die mit agonistischen, CD3 und CD28 spezifischen Antikörpern beschichtet sind.

Frisch isolierte CD4⁺CD25⁺ Treg-Zellen (2,5 x 10⁴ Zellen/Loch) wurden (A) in Mikrotiter platten inkubiert, die mit dem anti-CD3 Antikörper OKT3 und dem anti-CD28 Antikörper 15E8 beschichtet worden sind (Beschichtungskonzentrationen je 0,01-10 µg/ml), (B) in Mikrotiterplatten in Gegenwart des Antikörpers OKT3 und 15E8 löslich im Medium (je 0 - 10 µg/ml), (C) auf OKT3 Antikörper beschichteter Oberfläche (Beschichtungskonzentration 0,01 - 10 µg/ml) in Gegenwart von IL-2 (0 - 1.000 U/ml). Nach 6 Tagen wurde die Zellproliferation durch Markierung mit BrdU bestimmt. Der Ansatz wurde in Doppelproben durchgeführt, Mittelwerte der Doppelwerte sind gezeigt.

Fig. 4: Nachweis der Proliferation der Treg-Zellen auf beschichteten Oberflächen. Frisch isolierte CD4⁺CD25⁺ und CD4⁺CD25⁻ T-Zellen wurden mit CSFE markiert und 2.5 x 10⁴ Zellen/Loch in Gegenwart oder Abwesenheit von IL-2 (1.000 U/ml) auf Mikrotiterplatten inkubiert, die entweder mit OKT3 und 15E8 Antikörper (Beschichtungskonzentration je 10 µg/ml) oder OKT3 Antikörper allein (Beschichtungskonzentration 10 µg/ml) beschichtet worden sind, oder auf Mikrotiterplatten, die ausschließlich mit Beschichtungspuffer beschichtet worden sind. Nach 4 Tagen wurden die Zellen simultan gefärbt für CD25 und FoxP3 unter Verwendung des PE-konjugierten anti-CD25 und des APC-konjugierten anti-FoxP3 Antikörpers und mit Hilfe der Durchflusszytometrie analysiert. (A) zeigt die Expression von CD25 und FoxP3, (B) FoxP3 und CD25 Expression als mittlere Fluoreszenz Intensität (MFI). (C) Proliferation der CFSE-markierten FoxP3^{high} und FoxP3^{low} Zellen in der CD4⁺CD25⁻ und CD4⁺CD25⁺ T-Zell Population, die unter gleichen Bedingungen inkubiert worden waren. (D) Anzahl der proliferierenden FoxP3⁺ und FoxP3⁻ Zellen in der CD4⁺CD25⁻ und CD4⁺CD25⁺ T-Zell Population.

Fig. 5: Zytokin Sekretion der CD4⁺CD25⁺ und CD4⁺CD25⁻ T-Zell Populationen nach Inkubation auf beschichteten Oberflächen.

Frisch isolierte CD4⁺CD25⁺ und CD4⁺CD25⁻ T-Zellen (2,5 x 10⁴ Zellen/Loch) wurden 7 Tage in Gegenwart oder Abwesenheit von IL-2 (1.000 U/ml) in 95-Loch Mikrotierplatten inkubiert, die entweder mit dem Antikörper OKT3 und 15E8 (Beschichtungskonzentration je 10 µg/ml) oder OKT3 allein (Beschichtungskonzentration 10 µg/ml) oder mit Beschichtungspuffer beschichtet worden sind. Kulturüberstände für mit Hilfe eines ELISA für IFN-γ (A), IL-2 (B) und IL-10 (C) untersucht. Der Ansatz wurde in Dreifachansätzen durchgeführt und die Mittelwerte ± Standardabweichung des Mittelwertes berechnet.

Fig. 6: Amplifikation der CD4⁺CD25⁺ T-Zellen auf beschichteten Oberflächen.

Frisch isolierte CD4⁺CD25⁺ T-Zellen (1 x 10⁶ Zellen in 10 ml RPMI-1640-Medium, 10% FCS) wurden auf OKT3 und 15E8 Antikörper beschichteten Oberflächen (Beschichtungskonzentration je 10 µg/ml) kultiviert und die Zellzahl bestimmt. Nach Erreichen einer Zelldichte von 5 x 10⁶ Zellen/ml wurden 1 x 10⁶ T-Zellen in 10 ml auf eine neue beschichtete Oberfläche ausgesät.

Fig. 7: Amplifizierte Treg-Zellen behalten ihre Suppressorfunktion. Frisch isolierte CD4⁺CD25⁻ T-Zellen (2x10⁶/10 ml RPMI-1640-Medium, 10% FCS) wurden auf OKT3 und 15E8 Antikörper beschichteten Oberflächen (Beschichtungskonzentration je 10 µg/ml) 14 Tage kultiviert. Die Zellen wurden im Anschluss von der Platte genommen und 5 Tage in 20 ml RPMI-1640-Medium, 10% FCS ohne Aktivierung kultiviert. Frisch isolierte allogene CD3⁺T-Zellen wurden CFSE-markiert (1.25 µM) und zusammen (5 x 10⁴ Zellen/well) mit expandierten und voraktivierten CD4⁺CD25⁻ T-Zellen in RPMI-1640-Medium, 10% FCS mit und ohne lösliche OKT3 (10 µg/ml) und 15E8 (1 µg/ml) Antikörper für 6 Tage inkubiert und anschließend die Anzahl von nicht-proliferierten, CFSE⁺ T-Zellen bestimmt. Die Zahl der spezifisch proliferierenden, CFSE-markierten T-Zellen [%] wurde nach folgender Formel bestimmt: (1-Anzahl der CFSE⁺ T-Zellen ohne Aktivierung/Anzahl der CFSE⁺ T-Zellen mit Aktivierung)*100.

## Patentansprüche

1. Verfahren zur Amplifikation von regulatorischen T-Zellen (Treg-Zellen), umfassend das Inkubieren von Ausgangs-Treg-Zellen in Gegenwart wenigstens eines ersten agonistischen, stimulierenden Agens mit Spezifität für CD3 oder TCR und und wenigstens eines zweiten agonistischen, stimulierenden Agens mit Spezifität für CD28, wobei das erste und zweite agonistische Agens auf einer Oberfläche gebunden ist und das Inkubieren ohne IL-2 erfolgt.

2. Verfahren nach Anspruch 1, wobei das erste agonistisches Agens ein Agens mit Spezifität für CD3 ist.

3. Verfahren nach Anspruch 1, wobei das erste agonistisches Agens ein Agens mit Spezifität für TCR ist.

4. Verfahren nach einem oder mehreren der Ansprüche 1 bis 3, wobei das erste und/oder zweite agonistische Agens ein Antikörper ist, vorzugsweise beide Agentien Antikörper sind oder das erste und zweite agonistische Agens gemeinsam durch einen superagonistischen Antikörper dargestellt werden.

5. Verfahren nach einem oder mehreren der Ansprüche 1 bis 4, wobei das Inkubationsmedium RPMI-1640-Medium enthaltend fötales Kälberserum ist.

6. Verfahren nach einem oder mehreren der Ansprüche 1 bis 5, wobei die Oberfläche, an die das erste und zweite Agens gebunden sind, eine im Wesentlichen planare Oberfläche ist, wobei eine aktivierte Polymer-Oberfläche, insbesondere eine Polystyrol-Oberflächen oder eine Polystyrol-Mikrotiterplatte besonders bevorzugt ist.

7. Verfahren nach einem oder mehreren der Ansprüche 1 bis 6, das zur Amplifizierung humaner Treg-Zellen geeignet ist.

8. Verfahren nach Anspruch 7, wobei aus periphärem Blut isolierte Ausgangs-Treg-Zellen verwendet werden.

9. Im Wesentlichen planare Oberflächen, die mit wenigstens einem agonistischen für CD3 oder TCR spezifischen Agens und wenigstens einem agonistischen für CD28 spezifischen Agens beschichtet ist.

10. Verwendung einer beschichteten Oberfläche nach Anspruch 9 zur Amplifikation von regulatorischen T-Zellen.
